(19) Europäisches Patentamt
European Patent Office
Office européen des brevets

(11) Veröffentlichungsnummer: **0 074 077**
**B1**

# (12) EUROPÄISCHE PATENTSCHRIFT

(45) Veröffentlichungstag der Patentschrift: **19.06.85**

(51) Int. Cl.⁴: **C 07 D 233/14**, C 07 D 233/16, E 21 B 43/25, C 10 G 33/04, C 08 G 65/26, C 08 G 65/28

(21) Anmeldenummer: **82108037.1**

(22) Anmeldetag: **01.09.82**

## (54) Bisimidazoline, Verfahren zu deren Herstellung und deren Verwendung.

(30) Priorität: **05.09.81 DE 3135235**

(43) Veröffentlichungstag der Anmeldung: **16.03.83 Patentblatt 83/11**

(45) Bekanntmachung des Hinweises auf die Patenterteilung: **19.06.85 Patentblatt 85/25**

(84) Benannte Vertragsstaaten: **DE GB NL**

(56) Entgegenhaltungen:
**EP - A - 0 023 335**
**FR - A - 1 412 921**
**FR - A - 2 174 274**
**US - A - 3 246 008**
**US - A - 4 292 046**

(73) Patentinhaber: **HOECHST AKTIENGESELLSCHAFT, Postfach 80 03 20, D-6230 Frankfurt am Main 80 (DE)**

(72) Erfinder: **Ritschel, Werner, Dr., Gartenstrasse 17, D-6238 Hofheim am Taunus (DE)**
Erfinder: **Diery, Helmut, Dr., Theresenstrasse 45, D-6233 Kelkheim (Taunus) (DE)**
Erfinder: **Hille, Martin, Dr., In den Eichen 46, D-6237 Liederbach (DE)**

EP 0 074 077 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Bei der Förderung von Rohölemulsionen erfolgt bekanntlich eine zunehmende Verwässerung der geförderten Rohöle. Dieses mitgeförderte Wasser bildet mit dem Rohöl eine Wasser-in-Öl-Emulsion, wobei in dem einemulgierten Wasser Salze wie Natriumchlorid, Calziumchlorid und Magnesiumchlorid gelöst sein können. Außerdem sind in den Rohölemulsionen häufig Kohlensäure und Schwefelwasserstoff enthalten. All diese Substanzen bewirken Korrosionsschäden in den Fördereinrichtungen und in der Raffinerie, so daß allein schon aus diesem Grund das salzhaltige Wasser aus der Rohölemulsion mit Hilfe von Demulgatoren abgetrennt werden muß.

Ein Demulgator hat die Aufgabe, bei einer möglichst geringen Anwendungskonzentration die Emulsion zu brechen und bei diesem Separationsprozeß möglichst ohne Aufwendung oder mit minimaler zusätzlicher Wärme eine vollständige Wasserabscheidung zu bewirken und den Salzgehalt auf ein Minimum zu reduzieren. Die Qualitätskriterien für geliefertes Rohöl sind der Restgehalt an Salz und der Wassergehalt.

Die Rohöle sind je nach ihrer Provinienz unterschiedlich zusammengesetzt und die im Öl vorhandenen natürlichen Emulsionsstabilisatoren besitzen einen komplizierten und differenzierten chemischen Aufbau, so daß für jedes Öl gezielt Spalter entwickelt werden müssen. Bedingt durch unterschiedliche Förderungs- und Aufbereitungsbedingungen werden die Anforderungen, die an einen Demulgator gestellt werden, noch vielfältiger. Durch das ständige Erschließen neuer und Änderung der Förderbedingungen älterer Ölfelder bleibt daher die Entwicklung optimaler Demulgatoren für den jeweiligen Zweck eine akute Aufgabe.

Als nichtionische Demulgatoren für Erdölemulsionen sind bereits Umsetzungsprodukte von Alkylenoxid mit Alkylphenol-Aldehyd-Harzen bekannt (US-PS 2 499 368, 2 499 370, 2 560 333 und 2 574 543). Auch die Verwendung von Block- und Mischpolymerisaten aus Propylenoxid und Ethylenoxid für diesen Zweck ist bekannt (FR-PS 1 069 615 und DE-AS 1 018 179).

Es wurde nun gefunden, daß neue Bisimidazoline neben ihrer ausgezeichneten Wirkung als Demulgatoren für Rohöl auch gute Effekte als Korrosionsinhibitoren zeigen.

Gegenstand der Erfindung sind neue Bisimidazoline der Formel

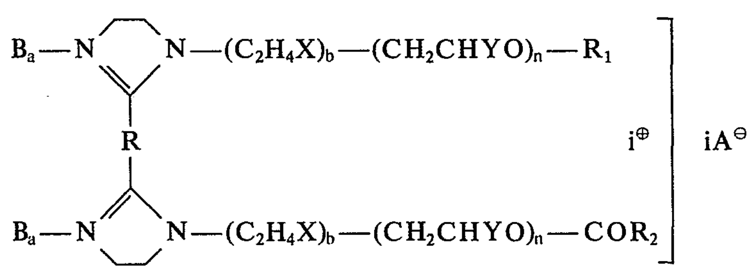

wobei R das Alkylgerüst einer dimerisierten Fettsäure mit 22 bis 42, vorzugsweise 34 C-Atomen, X ein Sauerstoffatom oder eine Gruppe der Formel

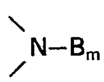

B Wasserstoff, Methyl, Ethyl, Benzyl oder eine Gruppe der Formel $-(CH_2CHYO)_n-R_1$,
Y Wasserstoff, Methyl oder Ethyl,
$R_1$ Wasserstoff oder eine Gruppe $-COR_2$,
$R_2$ $C_1-C_{22}$-, vorzugsweise $C_8-C_{22}$-Alkyl,
a 0 oder 1, b eine Zahl von 0 bis 4, m 1 oder 2, n eine Zahl von 0 bis 100, vorzugsweise 0 bis 20, i eine Zahl von 0 bis 2b + 2 und A ein Anion bedeutet, wie beispielsweise das Chlorid-, Bromid-, Methylsulfat-, Ethylsulfat- oder Dialkylphosphat-Ion, wobei b und n nicht gleichzeitig Null sein dürfen. Die oben aufgeführten Symbole X, B, Y, $R_1$, $R_2$, a, b, m, n können innerhalb einer Verbindung jeweils die gleiche oder voneinander verschiedene Bedeutungen haben.

Die Herstellung der Verbindungen der obigen Formel erfolgt, indem man zunächst eine dimerisierte Fettsäure der Formel II

$$HOOC-R-COOH \quad (II)$$

mit einem Di- oder Polyamin der Formel III

$$H_2N-CH_2CH_2-NH-(CH_2CH_2X)_bH \quad (III)$$

zu einem Bisimidazolin der Formel IV kondensiert,

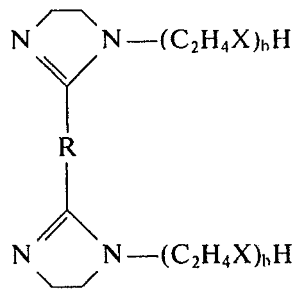

(IV)

dieses Bisimidazolin der Formel IV gegebenenfalls mit Ethylenoxid und/oder Propylenoxid bzw. Butylenoxid umsetzt, das erhaltene Reaktionsprodukt mit einer Säure der Formel V

$HOOC-R_2$ (V)

verestert und anschließend gegebenenfalls quaterniert oder neutralisiert.

Als dimerisierte Fettsäuren kommen vorzugsweise die Produkte in Frage, die unter den Bezeichnungen ®Pripol 1010, ®Pripol 1022 und Fatty Acid 7002 im Handel erhältlich sind. Diese Produkte können auch Anteile von trimeren oder höher kondensierten Fettsäuren enthalten. So enthält zum Beispiel Pripol 1022 ca. 20% trimere Anteile und Pripol 1010 nur ca. 3%. Diese dimerisierten Fettsäuren werden zunächst kondensiert mit zwei Mol eines Di- oder Polyamins der Formel III. Derartige Amine sind beispielsweise Aminoethylethanolamin, Ethylendiamin, Diethylentriamin, Triethylentetramin oder Pentaethylenhexamin. Die Kondensation kann erfolgen ohne Lösemittel in der Schmelze der Reaktionspartner oder in Gegenwart eines inerten Lösemittels bei der Siedetemperatur des Lösemittels. Als Lösemittel dient hier vorzugsweise Toluol oder Xylol, das gleichzeitig dazu dient, das gebildete Reaktionswasser zu entfernen.

Das bei dieser Kondensation entstandene Bisimidazolin der Formel IV kann dann, in Abhängigkeit von dem für das Symbol n gewählten Wert, nach bekannten Methoden oxalkyliert werden, vorzugsweise in Gegenwart eines basischen Katalysators wie Natriummethylat oder Natriumhydroxid. Als Alkylenoxide kommen vorzugsweise Ethylenoxid in Frage aber auch Mischungen von Ethylenoxid mit Propylenoxid oder Butylenoxid.

Diese Oxalkylierungsprodukte oder, wenn $n = 0$ ist, die Bisimidazoline der Formel IV werden dann mit einer oder mehreren Carbonsäuren der Formel V verestert. Als Carbonsäuren werden vorzugsweise $C_8$—$C_{22}$-Fettsäuren verwendet. Das Mengenverhältnis zwischen Carbonsäuren und Bisimidazolin der Formel IV bzw. dessen Oxalkylierungsprodukten kann so gewählt werden, daß eine oder mehrere Acylgruppen in dem Ester vorhanden sind.

Die Veresterung kann erfolgen mit reinen Carbonsäuren der Formel V oder mit Mischungen verschiedener derartiger Carbonsäuren. Analog zu der ersten Stufe kann auch hier die Reaktion in der Schmelze der Reaktionspartner durchgeführt werden bei Temperaturen von ca. 160—180°C oder in einem inerten Lösungsmittel wie oben beschrieben.

Die so erhaltenen Ester können dann noch quaterniert werden, entweder durch einfache Addition von Säuren, wie zum Beispiel Essigsäure, Schwefelsäure oder Phosphorsäure, für den Fall $B = H$ oder durch Umsetzung mit alkylierenden Reagenzien wie zum Beispiel Methylchlorid, Benzylchlorid, Dimethylsulfat oder Trimethylphosphat, vorzugsweise bei Temperaturen von 60—70°C in einem niederen Alkohol oder in Toluol.

Die so erhaltenen Produkte eignen sich sowohl in der quaternisierten wie auch in der teil- oder nichtquaternisierten bzw. neutralisierten Form sehr gut zum Demulgieren von Rohölemulsionen. Man setzt diese Produkte der Rohölemulsion in Konzentrationen von 2 bis 400, vorzugsweise 5 bis 50 ppm zu, entweder in unverdünnter Form oder als Lösungen, die im Verhältnis bis 1 : 200 mit einem organischen Lösungsmittel verdünnt wurden.

Die folgenden Beispiele sollen die Erfindung erläutern.

Allgemeine Arbeitsvorschrift zur Herstellung der Bisimidazoline IVa—c für die Beispiele 1—7

Am Wasserabscheider werden 0.5 Mol einer dimeren Fettsäure mit 1 Mol eines Amins in 300 g Xylol erhitzt, bis ca. 36 ml Wasser ausgekreist sind. Nach Abdestillieren des Xylols erhält man ein viskoses, noch gießbares Produkt.

Herstellung von IVa:

Nach der allgemeinen Arbeitsvorschrift aus 285 g (0.5 Mol) der dimeren Fettsäure »Pripol 1022« und 103 g (1 Mol) Diethylentriamin.

Herstellung von IVb:

Aus 285 g (0.5 Mol) »Pripol 1022« und 104 g (1 Mol) Aminoethylethanolamin.

Herstellung von IVc:
Aus 285 g (0.5 Mol) »Pripol 1010« und 103 g (1 Mol) Diethylentriamin.

Die Bisimidazoline IVa—IVc zeigen im IR-Spektrum die charakteristische C = N-Absorption bei 1610 $cm^{-1}$.

Beispiel 1

Zur Lösung des nach der allgemeinen Arbeitsvorschrift hergestellten Bisimidazolins IVa in 300 g Xylol werden 250 g (1 Mol) Stearinsäure zugegeben und weiter erhitzt, bis 18 ml $H_2O$ ausgekreist sind. Nach Abdestillieren des Xylols fügt man 650 g Isobutanol zu und quaternisiert in einem Autoklaven bei 70°C mit Methylchlorid. Man erhält eine braune, gießbare Flüssigkeit mit einem Gehalt von 50% der Verbindung der Formel

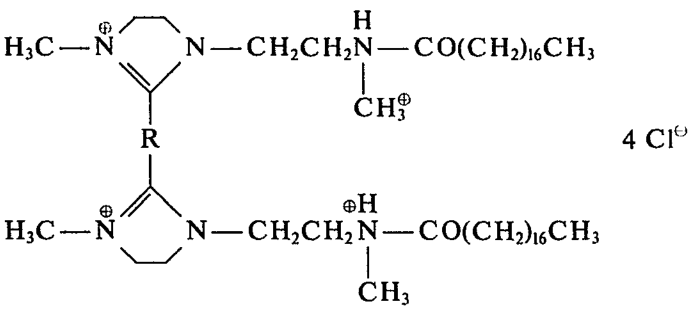

R bedeutet hier wie auch in den folgenden Formeln das $C_{34}$-Alkylgerüst einer dimerisierten Fettsäure.

Beispiel 2

Nach Umsetzung des Bisimidazolins IVa mit 250 g Stearinsäure wie in Beispiel 1 werden bei 60°C 60 g (1 Mol) Eisessig zugerührt. Nach Abdestillieren des Xylols und Zugabe von 650 g Isobutanol erhält man eine Lösung mit einem Gehalt von 50% der Verbindung der Formel

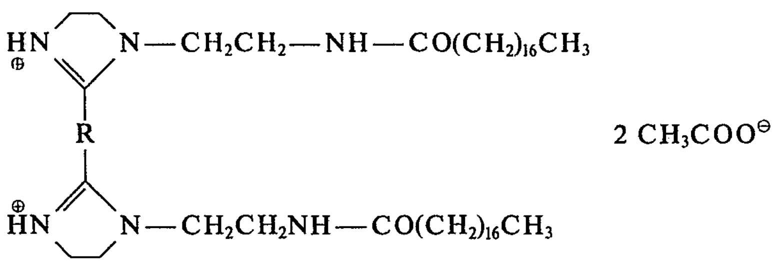

Beispiel 3

Zur Lösung des Imidazolins IVb in 300 g Xylol werden 125 g (0.5 Mol) Stearinsäure gegeben und am Wasserabscheider 9 ml $H_2O$ ausgeschleppt. Nach Abdestillieren des Xylols fügt man 520 g Isobutanol zu und setzt im Autoklaven bei 70°C mit Methylchlorid um, bis keine weitere Aufnahme erfolgt. Man erhält eine 50%ige Lösung in Isobutanol der Verbindung der Formel

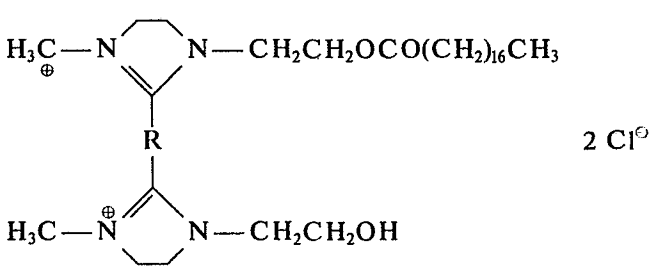

Beispiel 4

An das Bisimidazolin IVb werden in einem Autoklaven nach Zusatz von 1.5 g NaOH pulv. bei 160°C und 5 bar 220 g (5 Mol) Ethylenoxid addiert. Anschließend werden 125 g (0.5 Mol) Stearinsäure

zugegeben und in einer Destillationsapparatur auf 160—180°C erhitzt, bis 9 ml Wasser abdestilliert sind. Nach Zufügen von 730 g Isobutanol wird in einem Autoklaven bei 70°C mit Methylchlorid quaternisiert. Man erhält eine 50%ige Lösung in Isobutanol der Verbindung der Formel

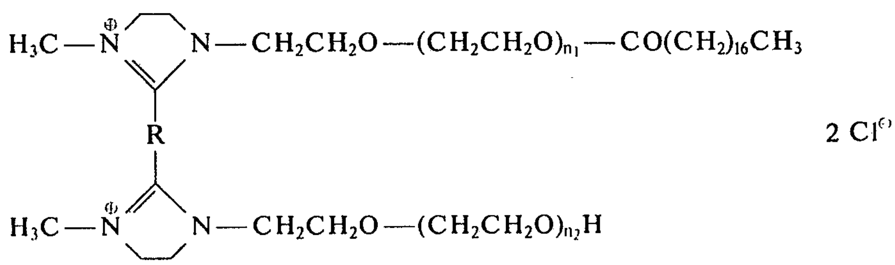

$n_1 + n_2 = 10$

Beispiel 5

An das Bisimidazolin IVb werden nach bekannter Weise in einem Autoklaven 660 g (15 Mol) Ethylenoxid addiert. Anschließend werden 250 g (1 Mol) Stearinsäure zugefügt und in einer Destillationsapparatur 18 ml $H_2O$ abdestilliert. Nach Zugabe von 1300 g Isobutanol wird mit Methylchlorid quaternisiert. Die entstandene Lösung enthält 50% der Verbindung der Formel

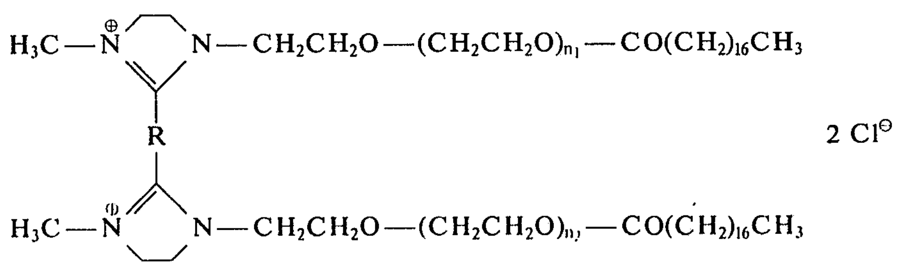

$n_1 + n_2 = 30$

Beispiel 6

An das Bisimidazolin IVc werden nach der üblichen Methode zunächst 220 g (5 Mol) Ethylenoxid und dann 280 g (5 Mol) Propylenoxid addiert. Dann werden 380 g (1.5 Mol) Talgfettsäure zugesetzt und in einer Destillationsapparatur erhitzt, bis 27 ml $H_2O$ abdestilliert sind. Man fügt 1200 g Isobutanol zu und erhält eine braune, gießbare Flüssigkeit, die 50% der Wirksubstanz der folgenden Formel enthält.

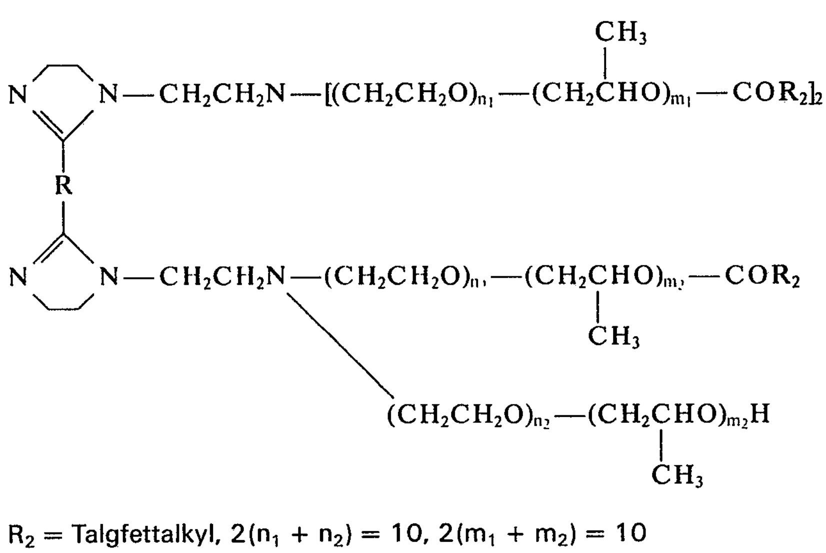

$R_2$ = Talgfettalkyl, $2(n_1 + n_2) = 10$, $2(m_1 + m_2) = 10$

Beispiel 7

An das Bisimidazolin IVa werden in üblicher Weise 110 g (2.5 Mol) Ethylenoxid addiert, anschließend wird mit 500 g (2 Mol) Talgfettsäure verestert. Nach Zugabe von 925 g Isobutanol erhält man eine 50%ige Lösung der Verbindung der Formel

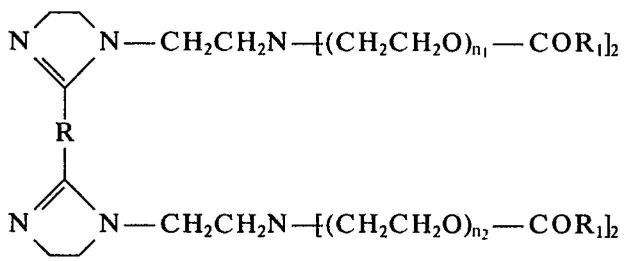

$R_1$ = Talgfettalkyl, $n_1 + n_2 = 5$

Beispiel 8

420 g (0.5 Mol) der dimeren Fettsäure »Fatty Acid 7002« werden mit 104 g (1 Mol) Aminoethylethanolamin ohne Lösungsmittel erhitzt, bis 36 ml $H_2O$ abdestilliert sind. Dann gibt man 72 g (0.5 Mol) 2-Ethylhexansäure zu und destilliert weitere 9 ml $H_2O$ ab. Nach Zufügen von 630 g Isobutanol wird mit 80 g Dimethylsulfat bei 65°C quaternisiert, wodurch man eine 50%ige Lösung (in Isobutanol) der Verbindung der folgenden Formel erhält.

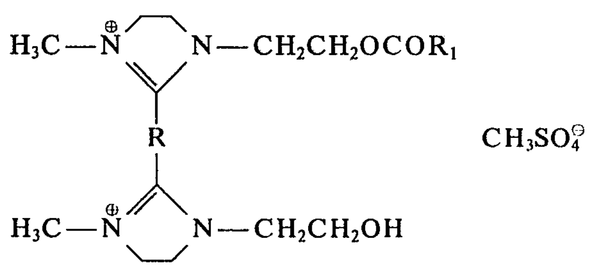

$R_1$ = 2-Ethylhexyl

In den folgenden Tabellen wird die demulgierende Wirkung der erfindungsgemäßen Verbindungen an Rohölemulsionen bei den in den Ölfeldern üblichen Bedingungen und Einsatzmengen aufgezeigt. Die Demulgatoren wurden dabei in 50%igen isobutanolischen Lösungen angewendet, die mit Mikrodosierungseinrichtungen injiziert werden. Die Abscheidung des emulgierten Wassers erfolgt in konischen, kalibrierten, mit Stopfen verschließbaren Gläsern und die Menge der Emulsion beträgt jeweils 100 $cm^3$. In den Tabellen werden die in bestimmten Zeiten abgeschiedenen Mengen Emulsionswasser in % angegeben. Durch Vorversuche wurde der absolute Wassergehalt der Emulsionen jeweils nach Dean-Stark bestimmt. Die Dosiermenge der Demulgatoren, der absolute Wassergehalt der Emulsion, die Separiertemperatur und der Emulsionsursprung sind in den einzelnen Tabellen aufgeführt.

Tabelle I

Demulgiertemperatur: 50°C
Wassergehalt der Emulsion: 43%
Dosiermenge: 40 ppm
Ursprung: Nordsee
Angabe: % Wasserseparation

| Verbindung aus Beispiel | Minuten 10 | 20 | 30 | 60 | 120 | 180 |
|---|---|---|---|---|---|---|
| 1 | 12 | 33 | 63 | 78 | 92 | 99 |
| 2 | 14 | 38 | 68 | 85 | 98 | 100 |
| 3 | 16 | 39 | 71 | 90 | 100 | 100 |
| 4 | 13 | 35 | 68 | 83 | 91 | 98 |
| 5 | 11 | 34 | 65 | 81 | 90 | 97 |
| 6 | 18 | 38 | 69 | 88 | 100 | 100 |
| 7 | 12 | 35 | 67 | 86 | 95 | 99 |
| 8 | 18 | 38 | 69 | 91 | 98 | 100 |
| ohne Zusatz | 0 | 0 | 0 | 0 | 0 | 0 |

Tabelle II

Demulgiertemperatur: 40°C
Wassergehalt: 65%
Dosiermenge: 50 ppm
Ursprung: Emsland
Angabe: % Wasserseparation

| Verbindung aus Beispiel | Minuten 10 | 30 | 60 | 120 | 180 |
|---|---|---|---|---|---|
| 1 | 12 | 33 | 62 | 78 | 93 |
| 2 | 21 | 54 | 88 | 96 | 100 |
| 3 | 25 | 62 | 93 | 100 | 100 |
| 4 | 17 | 48 | 74 | 98 | 100 |
| 5 | 16 | 38 | 74 | 92 | 98 |
| 6 | 24 | 54 | 83 | 98 | 100 |
| 7 | 20 | 48 | 83 | 94 | 100 |
| 8 | 22 | 43 | 76 | 91 | 97 |
| ohne Zusatz | 0 | 0 | 0 | 0 | 0 |

Tabelle III

Demulgiertemperatur: 52°C
Wassergehalt: 28%
Dosiermenge: 60 ppm
Ursprung: Borneo
Angabe: % Wasserseparation (W), % Interface (Sludge) (I)

| Verbindung aus Beispiel | Minuten | | | | | | | |
|---|---|---|---|---|---|---|---|---|
| | 20 | | 60 | | 120 | | 180 | |
| | % W | % I | % W | % I | % W | % I | % W | % I |
| 1 | 40 | 15 | 85 | 4 | 93 | 1,0 | 98 | 1,0 |
| 2 | 36 | 18 | 78 | 6 | 90 | 3,5 | 96 | 2,0 |
| 3 | 42 | 15 | 85 | 3 | 98 | 1,0 | 100 | 0 |
| 4 | 42 | 11 | 85 | 2,5 | 98 | 0,5 | 100 | 0 |
| 5 | 51 | 10 | 90 | 1,0 | 100 | 0 | 100 | 0 |
| 6 | 47 | 12 | 88 | 2,0 | 99 | 0,2 | 100 | 0 |
| 7 | 53 | 8 | 88 | 1,5 | 100 | 0 | 100 | 0 |
| 8 | 40 | 14 | 79 | 5 | 94 | 1,5 | 98 | 1,5 |
| ohne Zusatz | 0 | 100 | 0 | 100 | 0 | 100 | 0 | 100 |

Tabelle IV

Demulgiertemperatur: 48°C
Wassergehalt: 19%
Dosiermenge: 50 ppm
Ursprung: Nigeria
Angabe: % Wasserseparation (W), % Interface bez. auf Öl (I)

| Verbindung aus Beispiel | Minuten | | | | | | | |
|---|---|---|---|---|---|---|---|---|
| | 30 | | 60 | | 120 | | 180 | |
| | % W | % I | % W | % I | % W | % I | % W | % I |
| 1 | 55 | 8,0 | 88 | 2,0 | 96 | 1,0 | 99 | 0,5 |
| 2 | 58 | 6,0 | 91 | 1,5 | 99 | 0,5 | 100 | 0 |
| 3 | 50 | 10,0 | 97 | 2,5 | 100 | 0 | 100 | 0 |
| 4 | 53 | 7,0 | 96 | 1,5 | 100 | 0 | 100 | 0 |
| 5 | 61 | 6,5 | 98 | 1,0 | 100 | 0 | 100 | 0 |
| 6 | 58 | 7,0 | 97 | 1,0 | 100 | 0 | 100 | 0 |
| 7 | 55 | 7,5 | 90 | 1,5 | 99 | 1,5 | 99 | 0,5 |

Fortsetzung

| Verbindung aus Beispiel | Minuten | | | | | | | |
|---|---|---|---|---|---|---|---|---|
| | 30 | | 60 | | 120 | | 180 | |
| | % W | % I | % W | % I | % W | % I | % W | % I |
| 8 | 48 | 11,0 | 85 | 3,0 | 93 | 2,0 | 97 | 1,0 |
| ohne Zusatz | 0 | 100 | 0 | 100 | 0 | 100 | 0 | 100 |

Inhibierung der Korrosion

Zur Ermittlung der Korrosionsinhibitorwirkung wurden Teststreifen aus Kohlenstoffstahl mit einer Oberfläche von 20 $cm^2$ bei 60°C jeweils 6 Stunden lang in 20%ige Natriumchloridlösungen getaucht, die einen Zusatz von 10 mg/l, 20 mg/l bzw. 30 mg/l des zu prüfenden Produktes enthielten. Durch die Testlösungen perlte während der Prüfung ein ständiger Strom von Kohlenoxid. Der hiernach festgestellte absolute Gewichtsverlust der Metallstreifen diente als Maß für die Korrosiovität.

Die erhaltenen Ergebnisse sind in Tabelle V zusammengestellt.

Tabelle V

| Produkt gemäß Beispiel | Einsatzmenge, mg/l | | |
|---|---|---|---|
| | 10 | 20 | 30 |
| | Gewichtsverlust, mg | | |
| 1 | 2,6 | 2,6 | 2,8 |
| 2 | 8,5 | 6,2 | 5,5 |
| 3 | 3,3 | 3,1 | 2,8 |
| 4 | 21,0 | 14,0 | 7,0 |
| 5 | 23,0 | 14,0 | 7,5 |
| 6 | 20,0 | 11,0 | 6,3 |
| 7 | 7,9 | 4,6 | 3,0 |
| 8 | 4,8 | 3,3 | 2,7 |
| ohne Zusatz | – | 24,5 | – |

Die Untersuchungsergebnisse zeigen, daß die gemäß der Erfindung verwendeten Produkte die charakteristischen Eigenschaften eines guten Emulsionsspalters und gleichzeitig die eines Korrosionsinhibitors besitzen. Sie zeigen weiter, daß der Sludge bei den schwer spaltbaren Borneo- oder Nigeria-Rohölemulsionen bei der Verwendung dieser Produkte weitgehend reduziert wird.

**Patentansprüche**

1. Bisimidazoline der Formel

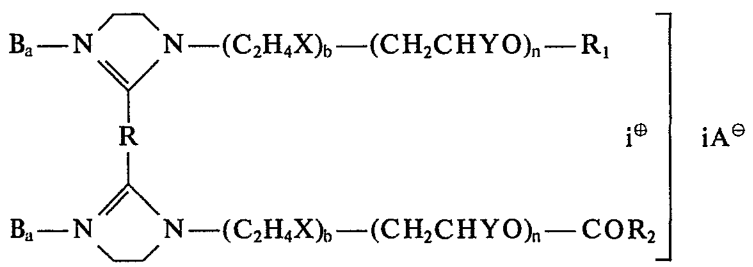

wobei R das Alkylgerüst einer dimerisierten Fettsäure mit 22 bis 42 C-Atomen, X ein Sauerstoffatom oder eine Gruppe der Formel

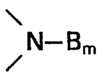

B ein Wasserstoff, Methyl, Ethyl, Benzyl oder eine Gruppe der Formel $-(CH_2CHYO)_n-R_1$,
Y Wasserstoff, Methyl oder Ethyl,
$R_1$ Wasserstoff oder eine Gruppe $-COR_2$,
$R_2$ $C_1-C_{22}$-Alkyl,
a 0 oder 1, b eine Zahl von 0 bis 4, m 1 oder 2, n eine Zahl von 0 bis 100, i eine Zahl von 0 bis 2b + 2 und A ein Anion bedeutet, wobei b und n nicht gleichzeitig Null sein dürfen.

2. Bisimidazoline nach Anspruch 1, wobei R das Alkylgerüst einer dimerisierten Fettsäure mit 34 C-Atomen bedeutet.

3. Bisimidazoline nach Anspruch 1, wobei $R_2$ $C_8-C_{22}$-Alkyl bedeutet.

4. Bisimidazoline nach Anspruch 1, wobei n eine Zahl von 0 bis 20 bedeutet.

5. Verfahren zur Herstellung der Bisimidazoline nach Anspruch 1, dadurch gekennzeichnet, daß man zunächst eine dimerisierte Fettsäure der Formel II

$$HOOC-R-COOH \quad (II)$$

mit einem Di- oder Polyamin der Formel III

$$H_2N-CH_2CH_2-NH-(CH_2CH_2X)_bH \quad (III)$$

zu einem Bisimidazolin der Formel IV kondensiert,

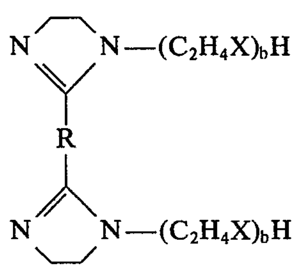

(IV)

dieses Bisimidazolin der Formel IV gegebenenfalls mit Ethylenoxid und/oder Propylenoxid bzw. Butylenoxid umsetzt, das erhaltene Reaktionsprodukt mit einer Säure der Formel V

$$HOOC-R_2 \quad (V)$$

verestert und anschließend gegebenenfalls quaterniert oder neutralisiert.

6. Verwendung der Bisimidazoline nach Anspruch 1 als Erdölemulsionsspalter.

**Claims**

1. A bisimidazoline of the formula

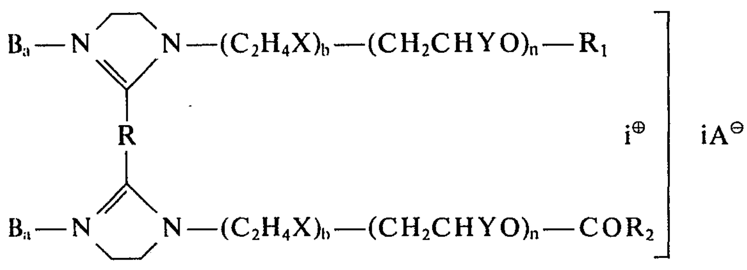

wherein R denotes the alkyl skeleton of a dimerized fatty acid having 22 to 42 C-atoms, X denotes an oxygen atom or a group of the formula

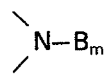

B denotes hydrogen, methyl, ethyl, benzyl or a group of the formula $-(CH_2CHYO)_n-R_1$,
Y denotes hydrogen, methyl or ethyl,
$R_1$ denotes hydrogen or a group $-COR_2$,
$R_2$ denotes $C_1-C_{22}$ alkyl,
a denotes 0 or 1, b denotes a number from 0 to 4, m denotes 1 or 2, n denotes a number from 0 to 100, i denotes a number from 0 to 2b + 2 and A denotes a anion.

2. A bisimidazoline according to claim 1, wherein R denotes the alkyl skeleton of a dimerized fatty acid having 34 C-atoms.

3. A bisimidazoline according to claim 1, wherein $R_2$ denotes $C_8-C_{22}$-alkyl.

4. A benzimidazoline according to claim 1, wherein n denotes a number from 0 to 20.

5. A process for the preparation of the bisimidazoline as claimed in claim 1, which comprises initially condensing a dimerized fatty acid of the formula II

$$HOOC-R-COOH \qquad (II)$$

with a diamine or polyamine of the formula III

$$H_2N-CH_2CH_2-NH-(CH_2CH_2X)_bH \qquad (III)$$

to give a bisimidazoline of the formula IV

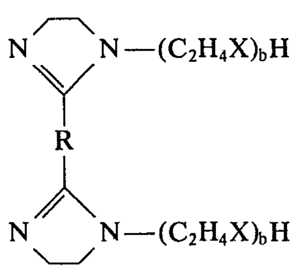

(IV)

reacting this bisimidazoline of the formula IV, if appropriate, with ethylene oxide and/or propylene oxide or butylene oxide, esterifying the reaction product obtained with an acid of the formula V

$$HOOC-R_2 \qquad (V)$$

and then, where appropriate, quaternizing or neutralizing.

6. The use of bisimidazolines as claimed in claim 1 as demulsifiers for crude petroleum emulsions.

**Revendications**

1. Bis-imidazolines répondant à la formule:

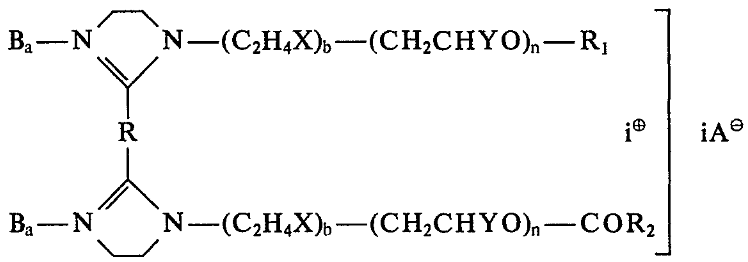

dans laquelle

R représente le squelette alkylique d'un acide gras dimérisé qui contient de 22 à 42 atomes de carbone,
X représente un atome d'oxygène ou un radical

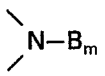

B représente l'hydrogène, un radical méthyle, éthyle ou benzyle ou un radical $-(CH_2CHYO)_n-R_1$,
Y représente l'hydrogène ou un radical méthyle ou éthyle,
$R_1$ représente l'hydrogène ou un radical $-COR_2$,
$R_2$ représente un radical alkyle en $C_1-C_{22}$,
a est égal à 0 ou à 1,
b représente un nombre de 0 à 4,
m est égal à 1 ou à 2,
n représente un nombre de 0 à 100,
i représente un nombre de 0 à 2b + 2,
A représente un anion,

et b et n ne peuvent être nuls en même temps.

2. Bis-imidazolines selon la revendication 1 dans lesquelles R représente le squelette alkylique d'un acide gras dimérisé contenant 34 atomes de carbone.
3. Bis-imidazolines selon la revendication 1, dans lesquelles $R_2$ représente un radical alkyle contenant de 8 à 22 atomes de carbone.
4. Bis-imidazolines selon la revendication 1, caractérisées en ce que n est un nombre de 0 à 20.
5. Procédé pour préparer des bis-imidazolines selon la revendication 1, procédé caractérisé en ce qu'on condense d'abord un acide gras dimérisé de formule II:

$$HOOC-R-COOH \qquad (II)$$

avec une diamine ou une polyamine de formule III

$$H_2N-CH_2CH_2-NH-(CH_2CH_2X)_bH \qquad (III)$$

de manière à obtenir une bis-imidazoline de formule IV:

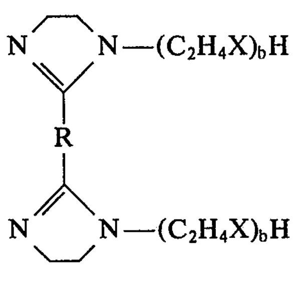

(IV)

on fait réagir éventuellement cette bis-imidazoline de formule IV avec l'oxyde d'ethylène et/ou l'oxyde de propylène ou l'oxyde de butylène, on estérifie le produit réactionnel obtenu avec un acide de formule V:

$$HOOC{-}R_2 \qquad (V)$$

et ensuite, le cas échéant, on quaternise ou on neutralise.

6. Application des bis-imidazolines selon la revendication 1 comme désémulsionnants pour produits pétroliers.